(19) Europäisches Patentamt
European Patent Office
Office européen des brevets

(11) Publication number : **0 392 665 B1**

(12) # EUROPEAN PATENT SPECIFICATION

(45) Date of publication of patent specification : **07.01.93 Bulletin 93/01**

(51) Int. Cl.$^5$ : **A61K 7/48,** A61K 7/06, C11D 1/34

(21) Application number : **90302698.7**

(22) Date of filing : **14.03.90**

(54) **Skin detergent composition.**

The file contains technical information submitted after the application was filed and not included in this specification

(30) Priority : **14.04.89 JP 94740/89**

(43) Date of publication of application : **17.10.90 Bulletin 90/42**

(45) Publication of the grant of the patent : **07.01.93 Bulletin 93/01**

(84) Designated Contracting States : **AT CH DE ES FR GB LI NL**

(56) References cited :
**EP-A- 0 037 161**
**EP-A- 0 224 796**
**DE-A- 3 432 227**
**GB-A- 2 027 047**

(56) References cited :
**PATENT ABSTRACTS OF JAPAN vol. 11, no. 365 (C-460)(2812) 27 November 1987, & JP-A-62 138418 (LION CORP.) 22 June 1987,**
**PATENT ABSTRACTS OF JAPAN vol. 8, no. 222 (C-246)(1659) 09 October 1984, & JP-A-59 106409 (ICHIMARU FUARUKOSU K.K.) 20June 1984,**

(73) Proprietor : **Kao Corporation**
**14-10, Nihonbashi Kayabacho 1-chome**
**Chuo-Ku Tokyo 103 (JP)**

(72) Inventor : **Kajihara, Yasushi**
**506. 6691-1, Kasukabe**
**Kasukabe-shi, Saitama (JP)**
Inventor : **Kaneda, Kenji**
**Kaoryo, 1-3 Asahigaokacho**
**Chiba-shi, Chiba (JP)**
Inventor : **Hirota, Hajime**
**31-9, Megurohoncho, 5-chome**
**Meguro-ku, Tokyo (JP)**

(74) Representative : **Bannerman, David Gardner et al**
**Withers & Rogers 4 Dyer's Buildings Holborn**
**London, EC1N 2JT (GB)**

EP 0 392 665 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention concerns a skin detergent composition and a method for its preparation. More particularly, it relates to a skin detergent composition that is comfortable to use and mild to the skin.

Heretofore, it has been recognized that phosphoric acid ester type surface active agents are non-irritating and extremely mild to the skin and they have therefore been utilized as one of ingredients for skin detergents. However, where a phosphoric acid ester type surface active agent is used as a skin detergent, water insoluble calcium salts are deposited upon rinsing, making rinsing difficult and impairing comfort.

On the other hand, water soluble chitin derivatives have been developed in recent years as moisture retaining agents in cosmetics and, as shown in Japanese Patent Publication Sho 62-49245 and Japanese Laid-Open Sho 62-138418, and as they show a moisture-retaining effect and are well absorbed onto the surface tissues of the skin or hair and also display a softening effect (conditioning effect) towards skin or hair, they have been utilized as one of the ingredients of shampoo compositions. However, concerning the use of water soluble chitin derivatives as ingredients for skin detergents, since rinsing off of the detergent is difficult as a result of their excellent absorption onto the skin, they are not comfortable to use and, accordingly, their application in skin detergents is difficult at present.

We have now surprisingly found that a skin detergent in which comfort in use, which is a problem with phosphoric acid ester type surface active agents, is remarkably improved and which has excellent rinsing properties, can be obtained by incorporating a chitin derivative, although this displays remarkable skin adsorption, at a concentration lower than that normally employed.

According to the present invention, we provide a skin detergent composition comprising (a) 5-95% by weight of one or more phosphoric acid ester type surface active agents represented by either or both of the following general formulae (I) or (II):

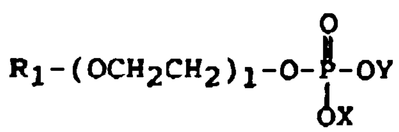

(I)

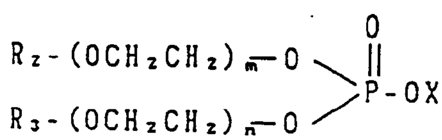

(II)

where each of $R_1$, $R_2$ and $R_3$ independently represents a saturated or unsaturated hydrocarbon group with 8 to 18 carbon atoms, each of X and Y independently represents hydrogen, an alkali metal, ammonium or an alkanol amine having a hydroxy alkyl group with 2 or 3 carbon atoms and each of l, m and n independently represents an integer from 0 to 10, and
(b) 0.0005 to 0.1% by weight of water soluble chitin derivative.

We also provide a method of preparing this composition by blending components (a) and (b) in the weight ratio set out above.

It is preferable that component (a) comprises either a compound of formula (I) or both a compound of formula (I) and a compound of formula (II), the weight ratio preferably being 10:0 to 5:5 and more preferably 10:0 to 7:3.

As the phosphoric acid ester type surface active agent of the ingredient (a), those with an added mol number of ethylene oxide groups from 0 to 3 are preferred and, in particular, those with no added ethylene oxide groups and having an alkyl group with 12 to 14 carbon atoms are particularly preferred. Specifically, there can be mentioned, for example, sodium mono- or di-lauryl phosphate, potassium mono- or di-lauryl phosphate, diethanolamine mono- or di-lauryl phosphate, triethanolamine mono- or di-lauryl phosphate, sodium mono- or di-myristyl phosphate, potassium mono- or di-myristyl phosphate, triethanolamine mono- or di-myristyl phosphate, etc.

The water soluble chitin derivative (component (b)) is preferably derived from chitin material present in the shells of crustacea such as crabs or shrimps, the outer skeletons of insects, or the cell walls of fungus or algae. As a typical example, there can be mentioned chitosan which is a deacetylation product of chitin.

As a specific method of obtaining chitosan, there can be mentioned, for example, a method of treating the shells of crabs or shrimps with dilute hydrochloric acid to remove the calcium carbonate and then treating the

resulting product with a dilute aqueous solution of sodium hydroxide to remove the protein, washing the resulting crude chitin with water, purifying if necessary, then deacetylating the chitin by means of an aqueous concentrated solution of sodium hydroxide, washing the thus-obtained crude chitosan with water and purifying if necessary.

Although chitin itself is generally water insoluble, chitin that has been treated by various methods may be water soluble. For example, a partially deacetylated water soluble chitin with a deacetylation degree of 40 to 60% in Japanese Patent Lid-Open No. Sho 53-47479 can be mentioned as an example. Water soluble deacetylated chitin can also be used in the present invention. Further, chitin derivatives rendered water soluble by the introduction of substituents, for example, carboxymethyl chitin, glycolated chitin, and chitin sulfate, may also be used in the present invention.

Although chitin is usually a high molecular weight material having a molecular weight greater than 1,000,000, the molecular weight is reduced in forming derivatives and derivatives tend to have molecular weights of about several hundreds of thousand.

The molecular weights of the water soluble chitin derivatives that may be used in the present invention can range from greater than 1,000,000, i.e. nearly as high as natural chitin, to as low as several tens of thousands.

For the skin detergent composition of the present invention, there is no particular restriction on the formulation and it may be formulated in any of the known forms as required. Examples are solid detergent, powdered detergent, granular detergent, paste detergent and liquid detergent.

The content of component (a) in the skin detergent composition according to the present invention ranges from 5 to 95% by weight, and preferably from 5 to 50% by weight, more preferably from 10 to 40% by weight in the case of a liquid detergent, preferably from 30 to 80% by weight, and more preferably from 40 to 70% by weight in the case of a paste detergent, and preferably from 60 to 95% by weight, and more preferably from 70 to 90% by weight in the case of a solid or powdered detergent. Further, the content of component (b) ranges from 0.0005 to 0.1% by weight. If the content of component (b) is less than 0.0005% by weight, its effect is insufficient. On the other hand, if it exceeds 0.1% by weight, rinsing is difficult and comfort in use is impaired. More preferably the content of component (b) is from 0.001 to 0.03% by weight.

In the skin detergent composition according to the present invention, various optional ingredients can be incorporated as required. As such optional ingredients, there can be mentioned, in addition to water, for example, foaming agents such as higher fatty acid salts, alkylamine oxide, fatty acid alkanolamides, imidazoline type amphoteric surface active agents, etc.; comfort improvers such as squalene and lanoline; inorganic and organic salts, diluents, perfumes, pigments, sterilizers, anti-inflammatory agents, moisture preservers, viscosity controllers, solubilizing agents, preservatives, and water soluble polymers.

The present invention will now be described in more detail by referring to the following non-limiting examples.

**Example 1**

The following liquid detergent composition was prepared, and the comfort in use and skin chapping were estimated respectively while varying the type and the amount of the water soluble chitin derivative.

Detergent composition

| | |
|---|---|
| Phosphoric acid ester type surface active agent | --- 30 parts by weight |
| Water soluble chitin derivative | --- 0.001-0.1 parts by weight |
| Ethanol | --- 5 parts by weight |
| Water | --- balance |

- Method of Evaluating the comfort in use -

After foaming the test specimen of the detergent manually by 20 panel members, they were rinsed off with

tap water and the comfort was evaluated by the panel members to obtain average values.

*Score Used

2 : easy to rinse
1 : somewhat easy to rinse
0 : neutral
-1 : somewhat difficult to rinse
-2 : difficult to rinse

- Method of Evaluating skin chapping -

An aqueous 15% solution of the test specimen for the detergent was prepared and the forearms of ten monitors were treated with the solution three times per day each time for 10 min., at intervals of three hours. The treatment was applied continuously for five days and the treated portions were judged with the naked eye to obtain average values as an index of skin chapping.

*Score for Skin Chapping

0 : with no skin chapping
1 : with slight skin chapping
2 : with medium skin chapping
3 : with heavy skin chapping

Results of the evaluation are shown in Table 1.

Table 1

| | | Phosphoric acid ester type surface active agent | Addition amount of water soluble chitin derivative (parts by weight | Score used | Skin chapping index |
|---|---|---|---|---|---|
| Comparative product | 1 | Triethanolamine monolauryl phosphate | ——— | -1.1 | 0.5 |
| | 2 | Triethanolamine monolauryl phosphate | Carboxymethyl chitin (0.500) | -1.3 | 0.2 |
| | 3 | Triethanolamine laurate | Carboxymethyl chitin (0.010) | 0.4 | 1.8 |
| Invented Product | 1 | Triethanolamine monolauryl phosphate | Carboxymethyl chitin (0.001) | 1.1 | 0.3 |
| | 2 | Triethanolamine monolauryl phosphate | Carboxymethyl chitin (0.010) | 1.6 | 0.2 |
| | 3 | Triethanolamine monolauryl phosphate | Carboxymethyl chitin (0.100) | 1.3 | 0.2 |
| | 4 | Triethanolamine monolauryl phosphate | Chitin Sulfate (0.010) | 1.0 | 0.2 |
| | 5 | Sodium monolauryl phosphate | Carboxymethyl chitin (0.010) | 1.3 | 0.2 |

Result

As has been described above, the detergent according to the present invention shows excellent performance in giving a refreshed feeling, satisfactory comfort and extremely low skin irritation.

On the other hand, if the content of the chitin derivative is greater as in Comparative Product 1, satisfactory comfort in use was not obtained since it was difficult to rinse away, making the skin feel slimy.

**Example 2 (Solid detergent)**

| | Parts by weight |
|---|---|
| Sodium monolauryl phosphate | 60 |
| Sodium dilauryl phosphate | 20 |
| Sodium lauryl phosphate | 10 |
| Carboxymethyl chitin | 0.006 |
| Perfume | 0.3 |
| Pigment | slight amount |
| | 0.3 |
| Water | balance |

With the composition of the above blend, a solid detergent mild to skin and giving excellent comfort in use could be obtained.

**Example 3 (Creamy Detergent)**

| | Parts by weight |
|---|---|
| Sodium monolauryl phosphate | 35 |
| Sodium monocetyl phosphate | 10 |
| Carboxymethyl chitin | 0.05 |
| Sodium chloride | 7 |
| Polyethylene glycol (molecular weight : 8000) | 5 |
| Sorbitol | 5 |
| Perfume | 0.7 |
| Water | balance |

With the composition of the above blend, a solid detergent mild to the skin and giving excellent comfort in use could be obtained.

## Claims

1. A skin detergent composition consisting essentially of (a) 5 to 95% by weight of at least one phosphoric acid ester having one or both of formulae (I), and (II)

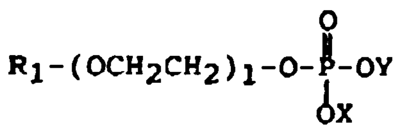

(I)

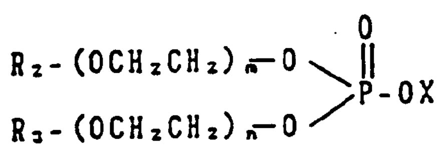

(II)

where each of $R_1$, $R_2$ and $R_3$ independently represents a saturated or unsaturated hydrocarbon group with 8 to 18 carbon atoms, each of X and Y independently represents hydrogen, an alkali metal, ammonium or an alkanol amine having a hydroxy alkyl group with 2 or 3 carbon atoms and each of l, m and n independently represents an integer from 0 to 10, and (b) 0.0005 to 0.1% by weight of a water-soluble chitin derivative.

2. The composition as claimed in Claim 1, which further includes a carrier.

3. The composition as claimed in Claim 1, in which either a component of formula (I) or both a component of formula (I) and a component of formula (II) in a weight ratio of 10:0 to 5:5.

4. A composition according to Claim 3 wherein the weight ratio is 10:0 to 7:3.

5. A composition according to any proceeding claim wherein the chitosan derivative is chitosan, partially-deacylated chitin, carboxymethyl chitin, glycolated chitin, or chitin sulfate.

6. A composition according to any preceding claim wherein the phosphate acid ester-type surface active agent has 0 to 3 moles of added ethylene oxide units.

7. A composition according to Claim 6, wherein the phosphate and ester-type surface-active agent has no added ethylene oxide groups and an alkyl group with 12 to 14 carbon atoms.

8. A method of preparing a skin detergent composition as claimed in claim 1, comprising blending (a) 5 to 95% by weight of at least one phosphoric acid ester having one or both of formulae (I) and (II), and (b) 0.0005 to 0.1% by weight of a water-soluble chitin derivative.

## Patentansprüche

1. Hautreinigungsmittel, bestehend im wesentlichen aus (a) 5 bis 95 Gew.%, wenigstens eines Phosphorsäureesters der Formeln (I) und (II):

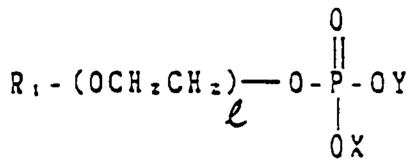

(I)

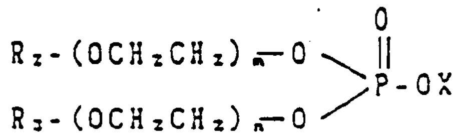

(II)

worin jedes $R_1$, $R_2$ und $R_3$ unabhängig eine gesättigte oder ungesättigte Kohlenwasserstoffgruppe mit 8 bis 18 Kohlenstoffatomen, jedes X und Y unabhängig Wasserstoff, ein Alkalimetall, Ammonium oder ein Alkanolamin mit einer Hydroxyalkylgruppe mit 2 oder 3 Kohlenstoffatomen und jedes $\ell$, m und n unabhängig eine ganze Zahl von 0 bis 10 bedeuten und (b) 0,0005 bis 0,1 Gew.% eines wasserlöslichen Chitinderivats.

2. Mittel nach Anspruch 1, das weiterhin einen Träger enthält.

3. Mittel nach Anspruch 1, enthaltend entweder eine Komponente gemäß Formel (I) oder sowohl eine Komponente gemäß Formel (I) und eine Komponente gemäß Formel (II) in einem Gewichtsverhältnis von 10:0 bis 5:5.

4. Mittel nach Anspruch 3, worin das Gewichtsverhältnis 10:0 bis 7:3 beträgt.

5. Mittel nach einem der vorhergehenden Ansprüche, wobei das Chitosanderivat Chitosan, teilweise deacetyliertes Chitin, glycoliertes Chitin oder Chitinsulfat ist.

6. Mittel nach einem der vorhergehenden Ansprüche, wobei das oberflächenaktive Mittel vom Phosphatsäureestertyp 0 bis 3 Mol zugegebene Ethylenoxideinheiten besitzt.

7. Mittel nach Anspruch 6, wobei das oberflächenaktive Mittel vom Phosphatsäureestertyp keine zusätzliche Ethylenoxidgruppen und eine Alkylgruppe mit 12 bis 14 Kohlenstoffatomen enthält.

8. Verfahren zur Herstellung eines Hautreinigungsmittels nach Anspruch 1, umfassend das Vermischen von (a) 5 bis 95 Gew.%, wenigstens eines Phosphorsäureesters gemäß einer oder beider Formeln (I) und (II) und (b) 0, 0005 bis 0,1 Gew.% eines wasserlöslichen Chitinderivats.

## Revendications

1. Composition détergente pour la peau constituée essentiellement de (a) 5 à 95 % en poids d'au moins un ester d'acide phosphorique ayant l'une des formules (I) et (II) ou les deux

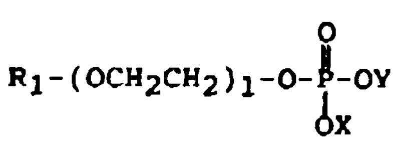

(I)

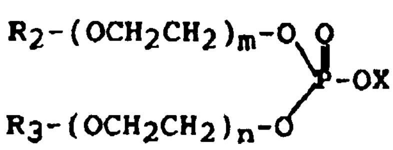

(II)

dans lesquelles chacun des $R_1$, $R_2$ et $R_3$ représente indépendamment un groupe hydrocarboné saturé ou

insaturé de 8 à 18 atomes de carbone, chacun des x et Y représente indépendamment l'hydrogène, un métal alcalin, l'ammonium ou une alcanolamine ayant un groupe hydroxyalkyle de 2 ou 3 atomes de carbone, et chacun des l, m et n représente indépendamment un entier de 0 à 10, et de (b) 0,0005 à 0,1 % en poids d'un dérivé de chitine soluble dans l'eau.

**2.** Composition telle que revendiquée dans la revendication 1, qui contient en outre un support.

**3.** Composition telle que revendiquée dans la revendication 1, qui comprend, soit un constituant de formule (I), soit à la fois un constituant de formule (I) et un constituant de formule (II) dans un rapport en poids de 10:0 à 5:5.

**4.** Composition selon la revendication 3 dans laquelle le rapport en poids est de 10:0 à 7:3.

**5.** Composition selon l'une quelconque des revendications précédentes, dans laquelle le dérivé de chitosane est le chitosane, une chitine partiellement désacylée, la carboxyméthylchitine, la chitine glycolée ou le sulfate de chitine.

**6.** Composition selon l'une quelconque des revendications précédentes, dans laquelle l'agent tensio-actif du type ester d'acide phosphorique a 0 à 3 unités d'oxyde d'éthylène fixées par addition.

**7.** Composition selon la revendication 6, dans laquelle l'agent tensio-actif du type ester d'acide phosphorique n'a pas de groupes oxyde d'éthylène fixée par addition et comporte un groupe alkyle de 12 à 14 atomes de carbone.

**8.** Méthode de préparation d'une composition détergente pour la peau telle que revendiquée dans la revendication 1, comprenant le mélange de (a) 5 à 95 % en poids d'au moins un ester d'acide phosphorique ayant l'une ded formules (I) et (II) ou les deux, et de (b) 0,0005 à 0,1 % en poids d'un dérivé de chitine soluble dans l'eau.